# EUROPEAN PATENT APPLICATION

(11) **EP 3 563 864 A1**
(43) Date of publication of application: **06.11.2019**
(21) Application number: 19162169.7
(22) Date of filing: 17.01.2014
(51) Int. Cl.: A61K 38/47, A61P 35/00, A61K 45/06, A61K 31/517, A61K 9/00, A61K 31/5377, A61K 31/404, A61K 31/506

(54) **SELECTIVE GLYCOSIDASE REGIMEN FOR IMMUNE PROGRAMMING AND TREATMENT OF CANCER**

(30) Priority: 18.01.2013 US 201361754056 P
(62) Divisional of application: 14740546.8
(71) Applicant: Kline, Ellis, Pendleton, South Carolina 29670-8839 (US)
(72) Inventor: Kline, Ellis, Pendleton, South Carolina 29670-8839 (US)
(74) Representative: Grund, Martin

(57) **Abstract**

The present invention relates to treatment and prevention of cancer with glycosidase(s). In various aspects the invention relates to the treatment and management of cancer to prevent metastasis or recurrence, or to improve outcome and rate of successful treatment with conventional therapeutic regimens. An enormous level of research and pharmaceutical development focuses on the treatment of cancer. Cancer remains one of the top targets of pharmaceutical pipelines. Products under development range from kinase inhibitors, to angiogenesis inhibitors, monoclonal antibodies against tumor targets, apoptosis inducers, anti-tumor vaccination, and conventional chemotherapeutic agents against various tumor targets and with various cytotoxic effects.

## Description

### PRIORITY

This Application claims priority to, and the benefit of, US Provisional Application No. 61/754,056, filed January 18, 2013, which is hereby incorporated by reference in its entirety.

### FIELD OF THE INVENTION

The present invention relates to treatment and prevention of cancer with an immune cascade elicited with a regimen of one or more glycosidase enzymes. In various aspects the invention relates to the treatment and management of cancer to prevent metastasis or recurrence, or to improve outcome and rate of successful treatment with conventional cancer therapies.

### BACKGROUND

An enormous level of research and pharmaceutical development focuses on the treatment of cancer. Cancer remains one of the top targets of pharmaceutical pipelines. Products under development range from kinase inhibitors, to angiogenesis inhibitors, monoclonal antibodies against tumor targets, apoptosis inducers, anti-tumor vaccination, and conventional chemotherapeutic agents against various tumor targets and with various cytotoxic effects. Many of these drugs are used in combination, and are effective for only a narrow range of patients or cancer types, some for only about 10% of the subject population of a particular cancer. See, New England J. Med. 2006, 355:2733-43; New England J. Med. 2004, 351:337-345. For this reason, and given the enormous costs of most cancer treatment regimens, a large industry has emerged focusing on tumor molecular or cellular analysis in the attempt to predict, with better accuracy, which patients might respond to certain treatments. Bin Wu et al., Predictive value of MTT assay as an in vitro chemosensitivity testing for gastric cancer: one institution's experience, World J Gastroenterol. 2008, May 21;14(19) 3064-3068. These molecular and cellular diagnostics remain themselves expensive, and their overall value in treating cancer remains a topic of debate. Further, some cancers are determined to be highly resistant or unlikely to respond to available active agents.

A treatment that is broadly applicable to cancer, or a particular type of cancer, is needed, to substantially improve outcomes with conventional therapies, or increase the number of patients that will respond to conventional therapies, as well as to reduce the need for reliance on experimental or poorly understood molecular or cellular tests to personalize treatment.

### SUMMARY OF THE INVENTION

The present invention provides compositions and methods for treating cancer and improving outcome in cancer treatment, including for drug resistant, recurrent, and/or metastatic cancers, as well as methods for treating early stage cancers. The treatment comprises *in vivo* administration of a glycosidase enzyme regimen (e.g., a regimen of one or more glycosidase enzymes) to the patient. In various embodiments, the glycosidase regimen is not targeted by the patient's immune system. In various embodiments, the glycosidase regimen provides one or more glycosidase enzymes active for removal of one or more terminal glycosyl groups on mammalian cells (e.g., cancer cells and/or immune cells) and/or serum proteins (e.g., group specific component). The glycosidase therapy reveals antigenic determinants on cancer cells, and elicits immune signaling cascades via its action on immune cells and/or serum proteins. Targeted terminal glycosyl groups may comprise, for example, sialosyl, beta-galactosyl, N-acetylgalactosamino, fucosyl, glucosyl, N-acetylglucosamino, and mannosyl residues, among others. Thus, the glycosidase regimen can include, in various embodiments, one or more of neuraminidase, galactosidase, N-acetylgalactosidase, fucosidase, glucosidase, N-acetylglucosaminidase, and mannosidase, among others. Without wishing to be bound by theory, the regimen increases immune signaling by removing effective amounts of glycosyl structures (e.g., sialic acid) from the surface of immune cells and/or serum proteins, as well as by unmasking cancer cells (which may have a preponderance of abberant glycosyl structures as compared to normal cells) such that the cancer cells can be more susceptible to immune surveillance. In this manner, the glycosidase regimen orchestrates or programs an effective immune response, allowing antigenic targeting of deleterious cells as well as eliciting proper levels of cytokine/chemokine cascades for therapy. In these or other embodiments, the glycosidase enzymes include at least one enzyme specific for a prominent terminal glycosyl residue (e.g., neuraminidase and/or galactosidase), and at least one enzyme specific for a prominent penultimate glycosyl residue (e.g., beta-galactosidase, fucosidase, or mannosidase) on the surface of cancer cells and/or immune cells. In some embodiments, such enzymes act synergistically with neuraminidase for cancer treatment. The regimen does not dysregulate (but instead coordinates) the patient's immune system, which is crucial in fighting cancer, and is effective even in the presence of certain levels of cytotoxic chemotherapies and/or radiation treatment, which have deleterious effects on immune cells. Further, the regimen is applicable for reducing or eliminating early stage cancers, while reducing or avoiding altogether surgical resection, radiation, or chemotherapy; or the regimen may be used for chronic therapy to prevent recurrence, or repeated therapy if recurrences or independent cancers develop, since the agent(s) are not targeted by the immune system in various embodiments. The regimen in various embodiments avoids excess removal of sialic acids or other glycosyl structures from normal cells so that they retain normal function.

The treatment regimen is broadly applicable across cancer types, or across the patient population for a particular type of cancer, and thus provides a high success rate. Thus, where 10 or more patients, or 20 or more patients, or 50 or more patients, or 100 or more patients are treated having the same type and stage of cancer, more predictable and improved outcomes are obtained. The treatment regimen need not depend on molecular tumor analysis or other chemosensitivity tool. In various embodiments, the treatment increases the rate of success with other treatments, including tumor resection, radiation treatment, and chemotherapy. For example, the glycosidase regimen in some embodiments enhances the performance status of patients, such that they are better able to tolerate conventional cancer therapy, and/or increases the therapeutic index of immunotoxic chemotherapies and radiation therapies. The treatment regimen in some embodiments reduces the invasiveness of cancer cells and/or reduces tumor volume, making the regimen useful with neoadjuvant therapy prior to surgery. The invention revolutionizes cancer treatment by rendering treatment less heterogeneous across cancer patients, and reducing the need for substantial personalized intervention.

In still other aspects, the invention provides a pharmaceutical composition comprising at least two of neuraminidase, galactosidase, N-acetylgalactosaminidase, fucosidase, glucosidase, N-acetylglucosaminidase, and mannosidase, and a pharmaceutically-acceptable excipient. For example, the composition may comprise neuraminidase and β-galactosidase. In some embodiments, the glycosidases are present at, collectively or individually, between about 10³ mg to 10⁻⁸ mg. The composition may be formulated for a variety of administration routes, including sublingual and parenteral delivery.

### DETAILED DESCRIPTION OF THE INVENTION

In one aspect, the invention provides a method for improving outcome or rate of response in cancer treatment. In this aspect, the invention comprises administering an *in vivo* regimen of one or more glycosidase enzymes to one or more patients undergoing treatment for cancer. The *in vivo* regimen stimulates immune signaling through removal of effective amounts of glycosides, notably sialic acids in some embodiments, from the surface of immune cells and/or serum proteins, unmasks cancer cells (also by the removal of effective amounts of glycosyl structures), and avoids excess removal of glycosides including sialic acid from normal cells. The regimen allows persistent antigenic targeting of the cancer cells by the elicited immune cascade. In some embodiments, the regimen comprises enzymes active for removal of glycosides that are prevalent on cancer cells, and which in various embodiments are terminal or penultimate glycosides. In some embodiments, the regimen comprises neuraminidase and a second glycosidase specific for the removal of a prevalent penultimate glycosyl residue, which can provide a synergistic treatment by avoiding, preventing, or slowing resialylation and/or re-capping of the glycosyl chains. The administration regimen, including as adjunct therapy and including embodiments that involve convenient patient dose monitoring, are as described in detail herein. According to this aspect, the effectiveness of the method does not critically rely on the molecular characteristics of the cancer or the patient's unique biology, unlike many conventional therapies. Thus, the method does not require that the patient(s) undergo a molecular analysis of the tumor or cancer cells prior to treatment, since cancer cells are broadly susceptible for being antigenically exposed by the glycosidase treatment.

Because many cancer therapies are targeted to cellular factors responsible for or involved in cellular growth or proliferation, and/or because cancers often develop drug resistance at these cellular determinants, cancer cells removed by biopsy are frequently subjected to molecular analysis of tumor markers. In some instances, a molecular profile of the tumor cells is generated to understand the potential sensitivity or resistance to available treatments. Exemplary molecular analyses include presence or overexpression of one or more of VEGF, PDGFRβ, CD31, HER2, PTEN, ERCC1, BRCA1, TOPO2α, Ki-67, P53, TS, ER, PR, or mutations in one or more of EGFR, ALK, KRAS, BRAF, and PI3K. The efficacy of the regimen described herein in various embodiments is not dependent on expression of mutation of a particular biomarker. Thus, in some embodiments, the method excludes molecular analysis of the rumor (e.g., for one or more of growth factor or growth factor receptor expression or over-expression, tumor suppressor protein expression, apoptosis marker expression, DNA repair protein expression, or kinase mutation) for predicting sensitivity or resistance to cancer therapies.

In these or other embodiments, the patient's tumor cells are not subjected to in vitro chemosensitivity analysis. A chemosensitivity assay is a laboratory test that measures the number of tumor cells that are killed by chemotherapy in vitro. Exemplary chemosensitivity (or "chemoresponse" assays) are described in US 2011/0238322, which descriptions are hereby incorporated by reference in its entirety. Since the glycosidase regimen has broad effectiveness against tumor cells, and in fact can increase the effectiveness of other chemotherapeutic agents as described herein, chemosensitivity testing is rendered less useful.

In other embodiments, the patient is determined to have a poor prognosis for survival with conventional therapy. For example, the prognosis may be an expected (e.g., greater than 50% chance of) survival of less than five years, less than three years, less than two years, or less than one year. The prognosis may be based on the type of cancer, including population response rates of the cancer type to radiotherapy and/or chemotherapy, and/or may be based upon a molecular characterization of the tumor cells, including expression levels of VEGF, PDGFRβ, CD31, HER2, PTEN, ERCC1, BRCA1, TOPO2α, Ki-67, P53, TS, ER, PR, or mutations in one or more of EGFR, ALK, KRAS, BRAF, and PI3K. Alternatively, prognosis may be based on a gene expression signature of the cancer that is indicative of chemotherapy resistance, likelihood of cancer recurrence, or a high risk group for survival. Gene expression signatures are becoming increasingly available for predicting tumor response to therapy and/or other classification of tumors for prognosis. Exemplary gene expression signatures are described in PCT/US2012/022594 (colon cancer), US 8,211,643 (NSCLC), US 2010-0331210 (breast cancer), US Patent 7,056,674, US Patent 7,081,340, US Patent 7,569,345, and US Patent 7,526,387, each of which is hereby incorporated by reference in its entirety. In some embodiments, the prognosis is based in part on the patient's condition and overall health or performance status (described in further detail below). Thus, in some embodiments, the glycosidase regimen is administered to patients with a poor prognosis with conventional therapies alone.

In some embodiments, the glycoside regimen is administered to patients with early stage cancer to substantially reduce or eliminate the need for surgery, radiation, or chemotherapy. For example, the regimen may be administered for from 1 to 4 months (e.g, from 1-2 months), and the patient then reevaluated for the need of conventional therapies, including resection or chemotherapy. Reevaluation generally includes tumor volume, blood panel (as described herein), malignancy markers, and patient's improvement in overall health or condition.

The glycosidase regimen in various embodiments is administered to patients with a higher performance status (e.g., at least 80%, or at least 70% using the Karnofsky scoring system) so as to prevent progression of the disease state, and enhance the patient's ability to accept chemotherapy and/or radiation treatment (or potentially avoid the need for such treatments). For example, in these embodiments, the patient at the time of initiating glycosidase therapy, is ambulatory and capable of self care. In still other embodiments, the glycosidase regimen is administered to patients with a low performance status (e.g., less than 50%, less than 30%, or less than 20% using the Karnofsky scoring system), so as to allow conventional radiotherapy and/or chemotherapy to be tolerated. In these embodiments, the patient at the time of initiating glycosidase therapy is largely confined to bed or chair and is disabled even for self-care. The glycosidase regimen described herein may have the ability to increase the patient's performance status (e.g., after from 1 to about 6 months of therapy, or from 1 to about 4 months of therapy) to allow for conventional chemotherapy treatment or radiation treatment, or improve the patient's ability to tolerate the treatment. In some embodiments, the glycosidase therapy is administered to patients that are deemed unfit for radiation or chemotherapy due to their advanced stage or performance status. In such embodiments, the glycosidase regimen enhances their fitness for these therapies.

In some embodiments, the patient is elderly, such as at least 65 years of age, or at least 70 years of age, or at least 75 years of age, and thus may be less fit for radiation and/or chemotherapy or surgery.

Performance status can be quantified using any system. Methods for scoring a patient's performance status are known in the art. The measure is often used to determine whether a patient can receive chemotherapy, adjustment of dose adjustment, and to determine intensity of palliative care. There are various scoring systems, including the Karnofsky score and the Zubrod score. Parallel scoring systems include the Global Assessment of Functioning (GAF) score, which has been incorporated as the fifth axis of the Diagnostic and Statistical Manual (DSM) of psychiatry. The Karnofsky score runs from 100 to 0, where 100 is "perfect" health and 0 is death. The score may be employed at intervals of 10, where: 100% is normal, no complaints, no signs of disease; 90% is capable of normal activity, few symptoms or signs of disease, 80% is normal activity with some difficulty, some symptoms or signs; 70% is caring for self, not capable of normal activity or work; 60% is requiring some help, can take care of most personal requirements; 50% requires help often, requires frequent medical care; 40% is disabled, requires special care and help; 30% is severely disabled, hospital admission indicated but no risk of death; 20% is very ill, urgently requiring admission, requires supportive measures or treatment; and 10% is moribund, rapidly progressive fatal disease processes. The Zubrod scoring system for performance status includes: 0, fully active, able to carry on all pre-disease performance without restriction; 1, restricted in physically strenuous activity but ambulatory and able to carry out work of a light or sedentary nature, e.g., light house work, office work; 2, ambulatory and capable of all self-care but unable to carry out any work activities, up and about more than 50% of waking hours; 3, capable of only limited self-care, confined to bed or chair more than 50% of waking hours; 4, completely disabled, cannot carry on any self-care, totally confined to bed or chair; 5, dead.

The methods described herein are applicable to a variety of cancers, including solid tumors and leukemias. In some embodiments, the cancer is one that is heavily sialylated. In various embodiments, the cancer is a soft-tissue sarcoma, squamous cell carcinoma, fibrosarcoma, myosarcoma, osteogenic sarcoma, angiosarcoma, endotheliosarcoma, or epithelial carcinoma. In some embodiments, the tumor histology is a serous adenocarcinoma, an endometroid adenocarcinoma, a mucinous adenocarcinoma, undifferentiated adenocarcinoma, transitional cell adenocarcinoma, or adenocarcinoma. Exemplary cancers include lung cancer including SCLC and NSCLC, mesothelioma, brain cancer, glioblastoma, head and neck cancer, esophageal cancer, breast cancer, lymphoma, prostate cancer, pancreatic cancer, liver cancer, stomach cancer, kidney cancer, colon or colorectal cancer, ovarian cancer, endometrial cancer, cervical cancer, testicular cancer, and melanoma. In still other embodiments, the cancer is a leukemia, such as chronic myelogenous leukemia (CML) or acute lymphoblastic leukemia (ALL). In some embodiments, the cancer is metastatic colorectal cancer. In some embodiments, the cancer is non-resectable liver or brain cancer. In some embodiments, where the cancer is a non-resectable solid tumor, the glycosidase regimen reduces and/or eliminates the tumor over time (e.g., at least 6 months or at least one year of therapy as described below)

The invention provides advantages that may differ depending on the type and stage of the cancer. For example, in some embodiments, the tumor has not invaded the underlying tissue, and the glycosidase regimen is effective to prevent progression of malignancy and prevent further invasiveness. In still other embodiments, the cancer has invaded the underlying tissue, but there is no local lymph node involvement or metastasis. In such embodiments, the glycosidase regimen prevents or slows continued invasion of the tissue. In other embodiments, there is involvement of local lymph nodes, but no metastatis to distant sites. In such embodiments, full metastasis is avoided or delayed with the glycosidase regimen. In still other embodiments, the treatment regimen helps to stimulate immune attack at multiple metastatic foci. Without wishing to be bound by theory, the regimen of the invention balances the benefits of direct in vivo deglycosylation of cancer cells, with complementary increase in immune signaling due to deglycosylation of immune cells and/or serum proteins). Since desialylation is kept within a beneficial threshold by dose and/or frequency of deglycosylation dosing, significant deleterious effects of substantial deglycosylation are avoided that could interfere with normal cellular functions. See Varki and Gagneux, Multifarious roles of sialic acids in immunity, Ann. N.Y. Acad. Sci. 1253:16-36 (2012). Proper in vivo deglycosylation (e.g. desialylation) is accomplished in some embodiments by a patient-adjusted dosing which is described in detail herein.

The stage of the cancer can determine the appropriate use of the glycosidase regimen in the context of patient care. For illustration, using the overall stage grouping, Stage I cancers are localized to one part of the body; Stage II cancers are locally advanced, as are Stage III cancers. Whether a cancer is designated as Stage II or Stage III can depend on the specific type of cancer; for example, in Hodgkin's Disease, Stage II indicates affected lymph nodes on only one side of the diaphragm, whereas Stage III indicates affected lymph nodes above and below the diaphragm. The specific criteria for Stages II and III therefore differ according to diagnosis. Stage IV cancers have often metastasized, or spread to other organs or throughout the body. Thus, in some embodiments, the cancer is stage I and is not locally advanced. In accordance with these embodiments, the glycosidase regimen prevents disease progression, and in some embodiments the cancer may be eliminated, in some embodiments without cytotoxic chemotherapy that can do more harm than good, or surgery. In some embodiments, the cancer is stage II or III, that is, the cancer may be locally advanced. In such embodiments, the invention helps prevent metastases and slow or stall cancer progression or reduce tumor volume and increase patient fitness, which renders chemotherapy, radiotherapy, and/or resection more effective. In still other embodiments, the cancer is stage IV, or is metastatic. In such embodiments, the regimen helps to control, reduce, or eliminate metastases, and to reduce the tumor burden. In each case, the glycosidase regimen may be provided in conjunction with other cancer therapies as described herein. In some embodiments, the glycosidase regimen helps to increase the patient's overall condition (e.g., performance status) such that chemotherapy and/or radiotherapy can be tolerated at the recommended dose.

In some embodiments, the cancer is non-resectable, and the glycosidase regimen is provided to prevent or delay progression of the disease. A non-resectable cancer is a malignancy which can't be surgically removed, due either to the number of metastatic foci, or because it is in a surgical danger zone. In some embodiments, the glycosidase regimen prepares the patient, and/or reduces tumor volume, prior to chemotherapeutic and/or radiation treatment, and may decrease the dose of chemotherapy or radiation required.

In some embodiments, the cancer is multidrug resistant. For example, the patient may have undergone one or more cycles of chemotherapy, without substantial response. Alternatively or in addition, the tumor has one or more markers of multidrug resistance. Such markers can include chemoresponse assays or molecular assays (including as already described). Thus, as used herein, the term "multidrug resistant" means that the cancer has exhibited non-responsiveness to at least one cycle of combination chemotherapy, or alternatively, has scored (diagnostically) as resistant to at least two of (including comparable agent to) docetaxel, paclitaxel, doxorubicin, epirubicin, carboplatin, cisplatin, vinblastine, vincristine, oxaliplatin, carmustine, fluorouracil, gemcitabine, cyclophosphamide, ifosfamide, topotecan, erlotinib, etoposide, and mitomycin. In such embodiments, the glycosidase regimen may increase the therapeutic window, rendering the chemotherapy more effective.

In some embodiments, the patient is in remission. For patients that achieve remission, whether through conventional cancer therapy (with or without glycosidase treatment), the glycosidase regimen preserves the period of remission. In such embodiments, these patients may be placed on chronic glycosidase treatment to avoid or delay recurrence. The chronic treatment, as described herein, may proceed for greater than one year, two years, five years or more, without substantially interfering with normal immune function.

In other embodiments, the cancer is a recurrence following conventional chemotherapy of the initial cancer. Often, recurrent cancer has developed drug resistance, and thus is particularly difficult to treat and often comes with a poor prognosis for survival. In such embodiments, the glycosidase regimen described herein, with or without other therapies, can extend survival and avoid unnecessary toxicity from ineffective chemotherapies for a better quality of life.

In some embodiments, the glycosidase regimen is administered to prepare the patient for radiation therapy, or in other embodiments, the glycosidase regimen is administered after radiation therapy to help the patient recover from radiation therapy. Alternatively still, the glycosidase regimen may be administered during the radiation treatment regimen to enhance the outcome, and/or reduce side effects. In some embodiments, the regimen described herein prevents or delays side effects of chemo and/or radiation therapy selected from candidiasis, lymphadenopathy, herpetic infections, deep fungal infections, bacterial infections, malnutrition, dehydration, xerostomia, and oral mucositis (OM). The regimen in some embodiments reduces the need for breaks and/or interruptions in chemotherapy and/or radiation therapy and refusal of the radiation therapy and/or chemotherapy treatment regimen. In certain embodiments, the present invention prevents or delays the onset of a condition selected from erythema, edema, ulcerations, hyperkeratosis, and pseudomembranous mucosa in the oral cavity of a patient.

In some embodiments, the glycosidase regimen provides for preventing or delaying the onset of oral mucositis. Oral mucositis (OM), or stomatitis, is a common and debilitating complication of cancer chemotherapy and radiation therapy. OM is an inflammatory response of the oral mucosa and intraoral soft tissue structures in the oral cavity that occurs in response to the administration of radiation therapeutics and chemotherapeutics, as well as other cytotoxic therapies. It typically affects the inner surfaces of the cheeks and lips, the floor of the mouth, the lateral surfaces of the tongue and the bottom surfaces of the tongue and the soft palate. Lesions can also occur on the hard palate and upper surface of the tongue. Specifically, OM results from the systemic effects of stomatotoxic chemotherapy agents and from the local effects of radiation directed to the oral mucosa or the oral cavity. Mucositis can limit the patient's ability to tolerate the full regimen of chemotherapy or radiotherapy, thereby impacting the effectiveness of the treatment. Further, patients with damaged oral mucosa and reduced immunity resulting from chemotherapy and radiotherapy are also prone to opportunistic infections in the mouth. It is therefore critical that OM be prevented or reduced as much as possible. Thus, in accordance with these aspects of the invention, the patient is more able to complete the planned course of therapy, by maintaining a sufficient nutritional state, and by avoiding the significant pain and discomfort associated with OM.

In some embodiments, the patient receives radiation therapy for head and neck cancer. For example, the cancer may be a squamous cell carcinoma of the oral cavity, oropharynx, hypopharynx or larynx. In other embodiments, the head and neck cancer is a salivary gland tumor, lymphoma or sarcoma. The patient may require radiation surgery at one, or a plurality of oral sites. Radiation therapy targeting such tumors, has the effect of (conventionally) inducing OM.

In addition to or as an alternative to such radiation therapy, in some embodiments the patient may receive a chemotherapy regimen, such as a chemotherapy that conventionally induces mucositis (stomatotoxin). The chemotherapy may be, for example but is not limited to, paclitaxel, doxorubicin, mithramycin, docetaxel, platinum-based chemotherapeutics (including but not limited to cisplatin and carboplatin), mitomycin, methotrexate, fluorouracil, 5-fluorouracil (5-FU), vinorelbine, topotecan, irinotecan, bleomycin, bleomycin hydrorxyurea, mitomycin, actinomycin, topoisomerase I and II inhibitors, anthracylines, epirubicin, idarubicin, mitoxantrone, valrubicin, etoposide, teniposide, rubitecan, and derivatives thereof. The chemotherapy may include a taxane and/or an antimetabolite and/or derivatives thereof.

Patients undergoing conventional radiation therapy to the head and neck typically experience erythema and mouth soreness within about two weeks of beginning therapy and often develop more severe damage to the oral epithelium within the following two weeks. When both chemotherapy and radiotherapy are administered, the incidence and severity of oral mucositis can be exacerbated.

The glycosidase regimen may be administered in preparation for radiation therapy, administered with radiation therapy, or to recuperate after radiation therapy. The radiation therapy may include External beam therapy (EBT) or Intensity-modulated radiation therapy (IMRT). EBT delivers a beam of high-energy x-rays to the location of the tumor. The beam is generated outside the patient (usually by a linear accelerator) and is targeted at the tumor site. These x-rays can destroy the cancer cells and careful treatment planning allows the surrounding normal tissues to be spared. No radioactive sources are placed inside the patient's body. IMRT is an advanced mode of high-precision radiotherapy that utilizes computer-controlled x-ray accelerators to deliver precise radiation doses to a malignant tumor or specific areas within the tumor. The radiation dose is designed to conform to the three-dimensional (3-D) shape of the tumor by modulating, or controlling, the intensity of the radiation beam to focus a higher radiation dose to the tumor while minimizing radiation exposure to healthy cells. Brachytherapy can also be employed, which uses sealed radioactive sources implanted into the treatment area which can be either temporary or permanent.

Typically, radiation treatments are given once or twice a day, about five days a week for five to seven weeks. In certain embodiments of the invention, the course of radiation therapy is from 3 to 10 weeks in duration. The course of radiation therapy may be about five to about seven weeks in duration. The course of radiation therapy may involve radiation treatment from about 5 to about 15 times per week. The course of radiation therapy may involve radiation treatment from about 7 to about 10 times per week. The patient may receive radiation therapy once or twice per day, at least 5 days per week, for from five to about seven weeks.

Where head and neck cancer are concerned, the course of radiation therapy may involve a cumulative dose of at least about 30 Gy to at least one oral site. In some embodiments, the course of radiation therapy is a cumulative dose of at least about 40 Gy, about 50 Gy, about 60 Gy, or about 70 Gy to at least one oral site. Thus, the course of radiation therapy may involve a cumulative dose of from about 50 to about 75 Gy to at least one oral site. In such embodiments, a single daily fraction is from about 1.5 Gy to about 2.5 Gy to at least one oral site. Of course the daily fraction and cumulative dose may be directed to two or more oral sites, including 3 or 4 oral sites.

In some embodiments, the patient receives both chemotherapy and radiation therapy. The chemotherapy may involve a stomatotoxic compound, including but not limited to a taxane, antimetabolite, and/or antibiotic. In various embodiments, the chemotherapy includes administration of cisplatin, fluorouracil, carboplatin, and/or paclitaxel. Where the chemotherapy comprises cisplatin, the patient may receive the cisplatin at about 80 to about 100 mg/m² from two to about four times per month. For example, the patient may receive about 80 to about 100 mg/m² of cisplatin approximately tri-weekly (once every three weeks). Where cisplatin is administered in approximately weekly doses, the dose may be in the range of about 30 to 40 mg/m².

In other embodiments, chemotherapy may be given during the course of radiation therapy, since such may be more effective than if given before a course of radiation therapy. In particular, where the cancer is advanced (advanced stage III or stage IV) the radiation treatment schedules sometimes include chemotherapy. Drugs most commonly given in conjunction with radiation therapy are cisplatin (Platinol) and Cetuximab (Erbitux). Occasionally, other drugs may include fluorouracil (5-FU, Adrucil), carboplatin (Paraplatin), and paclitaxel (Taxol). The chemotherapy may be given in a variety of ways, including a low daily dose, a moderately low weekly dose, or a relatively higher dose every three to four weeks.

The regimen of glycosidase may involve doses on each day of radiation treatment, or (approximately) before each radiation treatment, and/or during a course of chemotherapy administration. In certain embodiments, a dose of glycosidase is also administered on days in which no radiation and/or chemotherapy is administered, to maintain the relatively constant level of glycosidase signaling. In some embodiments, the glycosidase regimen is initiated prior to the start of radiation and/or chemotherapy treatment (e.g., for about two weeks, or about one month, leading up to the start of radiation and/or chemotherapeutic treatment), when the patient's immune system is substantially normal. In these embodiments, where the glycosidase treatment continues throughout the therapy, the patient's immune system is better able to withstand the radiation and/or chemotherapy assault.

Because the glycosidase regimen reduces some side effects and toxicity associated with chemotherapy and/or radiation therapy, and has the effect of improving the overall status of the patient, in some embodiments, the patient does not need a gastrostomy feeding tube during the course of radiation therapy. In some embodiments, the patient is not administered an opioid during at least the first 3 weeks of radiation therapy, or during at least the first 5 weeks of radiation therapy, or is not administered an opioid during the course of radiation therapy. Opioids include drugs such as buprenorphine, codeine, fentanyl and morphine. In various embodiments, the planned course of radiation therapy and/or chemotherapy is not disrupted due to toxicity or patient discomfort. In other embodiments, the regimen reduces the number and/or frequency of hospital and/or clinic visits due to discomfort or complications of therapy. In still other embodiments, the methods reduce the need for breaks and/or interruptions in chemotherapy and/or radiation therapy. In yet other embodiments, the methods of the present invention reduce refusal of the radiation therapy and/or chemotherapy treatment regimen.

In some embodiments, the glycosidase regimen is administered prior to tumor resection, optionally with neoadjuvant chemotherapy. Thus, in certain embodiments, the patient has not had surgery to remove cancerous tissue, but will receive chemotherapy to shrink and/or downgrade the tumor prior to any surgery. The term "neoadjuvant chemotherapy" means chemotherapy administered to cancer patients prior to surgery. Types of cancers for which neoadjuvant chemotherapy is commonly considered include, for example, breast, colorectal, ovarian, cervical, bladder, and lung. In certain embodiments, the patient is a breast cancer patient that will receive neoadjuvant chemotherapy. In such embodiments, the glycosidase treatment regimen (given with or prior to neoadjuvant therapy) enhances effectiveness of the neoadjuvant therapy, meanwhile helping the patient maintain immune function and readiness for surgical intervention (if necessary). In these embodiments, the glycosidase regimen may be provided for 1 to 4 weeks prior to neoadjuvant chemotherapy. Of course, in various embodiments described herein, the glycosidase regimen is the sole neoadjuvant therapy to reduce tumor volume prior to surgery. In these embodiments, the regimen helps achieve an optimal debulking of the tumor.

In still other embodiments, the glycosidase regimen is administered after tumor resection, optionally with adjuvant chemotherapy. In such embodiments, the glycosidase regimen increases the likelihood of successful therapy, or increases the time to recurrence. In some embodiments, the glycosidase regimen is administered to patients with optimal debulking status. In other embodiments, the glycosidase regimen is administered to patients with suboptimal debulking status. The debulking status means the reduction of tumor size due to surgery or radiation treatment. Debulking status may be scored categorically, for example, as optimal or sub-optimal. An optimal score includes patients in which the residual disease after radiation and/or surgery is less than about 1 cm. A suboptimal score includes patients in which the residual disease after radiation and/or surgery is greater than about 1 cm. After debulking, and particularly where debulking status is not optimal, the glycosidase regimen helps to prevent substantial tumor re-growth or progression of disease, including where chemotherapy or radiation therapy is provided after surgery.

In addition to the glycosidase regimen, the patient(s) may receive a. chemotherapy selected from an anthracyclin, taxol or taxoid, vinca alkaloid, alkylating agent, intercalating agent, kinase inhibitor, or nitrogen mustard. Exemplary agents include one or more of a topoisomerase inhibitor (I or II), apoptosis inducer, protease inhibitor, microtubule inhibitor, mitotic inhibitor, an antimetabolite, signal transduction inhibitor, estrogen receptor inhibitor, EGFR inhibitor, Her2 inhibitor, or an aromatase inhibitor.

Exemplary chemotherapeutic agents include one or more of daunorubicin, doxorubicin, epirubicin, idarubicin, adriamycin, vincristine, carmustine, cisplatin, 5-fluorouracil, tamoxifen, prodasone, sandostatine, mitomycin C, foscarnet, paclitaxel, docetaxel, gemcitabine, fludarabine, carboplatin, leucovorin, tamoxifen, goserelin, ketoconazole, leuprolide flutamide, vinblastine, vindesine, vinorelbine, camptothecin, topotecan, irinotecan hydrochloride, etoposide, mitoxantrone, teniposide, amsacrine, merbarone, piroxantrone hydrochloride, methotrexate, 6-mercaptopurine, 6-thioguanine, cytarabine (Ara-C), trimetrexate, acivicin, alanosine, pyrazofurin, pentostatin, 5-azacitidine, 5-azacitidine, 5-Aza-5-Aza-2'-deoxycytidine, adenosine arabinoside (Ara-A), cladribine, ftorafur, UFT (combination of uracil and ftorafur), 5-fluoro-2'-deoxyuridine, 5-fluorouridine, 5'-deoxy-5-fluorouridine, hydroxyurea, dihydrolenchiorambucil, tiazofurin, oxaliplatin, melphalan, thiotepa, busulfan, chlorambucil, plicamycin, dacarbazine, ifosfamide phosphate, cyclophosphamide, pipobroman, 4-ipomeanol, dihydrolenperone, spiromustine, geldenamycin, cytochalasins, depsipeptide, 4'-cyano-3-(4-(e.g., Zoladex) and 4'-cyano-3-(4-fluorophenylsulphonyl)-2-hydroxy-3-methyl-3'-(trifluoromethyl)propionanilide.

In some embodiments, the patient(s) receive one or more of anti-Her2/neu antibodies such as Herceptin, an anti-EGFR antibody such as Erbitux, a growth factor receptor antibody such as Avastin, a small molecule inhibitor such as Tarceva, Iressa, or sunitinib), or anti-CD20 such as Rituxan. In still other embodiments, the patient(s) receive erlotinib, gefitinib, lapatinib, cetuximab, panitumumab, or imatinib.

The chemotherapy in some embodiments is an stomatotoxic chemotherapy, such as one or a combination of 5-fluorouracil, methotrexate, cytarabine, cisplatin, carboplatin, paclitaxel, docetaxel, doxorubicin, and etoposide.

In still other embodiments, the glycosidase regimen is administered as an alternative to chemotherapy. In such embodiments, the patient may be a poor candidate for chemotherapeutic treatment, either due to the presence of molecular markers of drug resistance, or other risk factors such as the patient's condition.

Where chemotherapy is administered, the glycosidase regimen may be administered prior to a chemotherapy cycle, between chemotherapy cycles, or after the completion of chemotherapy. In some embodiments, the glycosidase regimen is initiated prior to the start of chemotherapy, with the regimen continuing throughout the chemotherapy cycle.

In some embodiments, the patient receives a bone marrow transplant, and the glycosidase regimen is provided prior to, and/or after transplant, to help prepare the patient for the procedure or to help the immune system recover.

In still other embodiments, the patient does not receive any additional immune stimulating agent (e.g., other than glycosidase therapy), to avoid inducing immune attack of the glycosidase itself. For example, in some embodiments, cancer vaccines or immune adjuvants or immune stimulants (including herbal stimulants and vitamin C supplements) are avoided within about 1 or 2 weeks of glycosidase administration.

In still other embodiments, the glycosidase treatment is administered in conjunction with a cancer vaccine, which may be a dendritic cell vaccine. Antigen-presenting cells (APCs) are critical for the antigen-specific priming of T cells, and dendritic cells (DCs) are the most potent stimulatory APCs. DCs constitute a heterogeneous population of cells, and they are able to differentiate from both bone marrow (BM) and peripheral blood precursors. DCs exhibit at least five important characteristics for the generation of T-cell-mediated anti-tumor immunity. A number of tumor-associated antigens have been identified as potential immunogens in DC-based vaccination strategies. Such tumor rejection antigens derive, for example, from oncogenes (ras), overexpressed genes (HER-2/neu), embryonic genes (MAGE, BAGE, GAGE), normal differentiation genes (MART-1/ Melan-A, gp100, tyrosinase), viral genes (HPV), tumor-suppressor genes (p53), B-cell idiotypes, and other tumor-associated proteins (PSMA, MUC1). The glycosidase regimen described herein can improve the success of antitumor vaccination, especially with vaccinations that require tumor antigen presentation. However, in still other embodiments, the glycosidase therapy is administered either before immunotherapy (e.g., within no more than one week of cancer immunotherapy) or after immunotherapy (e.g., within no more than one week), but is not administered simultaneously with immunotherapy, so as to avoid an immune reaction against the glycosidase enzyme(s).

Thus, in some embodiments, the glycosidase therapy is provided alongside cancer immunotherapy, including immunostimulator or immune checkpoint inhibitor. For example, the glycosidase therapy may be administered prior to, during, or after treatment with CTLA-4-Ig (abatacept).

In some aspects, the invention provides a method of preventing cancer in a patient predisposed to develop cancer, comprising, administering an immunotolerant glycosidase regimen to the patient. In some embodiments, the patient is characterized by genetic predisposition for cancer, which is optionally breast cancer, colon cancer, or skin cancer. For example, the patient may have at least two immediate family members that were afflicted with cancer, or the patient has had at least two prior incidences of cancer, but is cancer free at the time glycosidase therapy is initiated.

The glycosidase regimen provides one or more glycosidase enzymes active for removal of one or more terminal and/or penultimate glycosyl groups on mammalian cells (e.g., cancer cells and/or immune cells). Such terminal and penultimate glycosyl groups include, for example, sialosyl, galactosyl, N-acetylgalactosamino, fucosyl, glucosyl, N-acetylglucosamino, and mannosyl residues. Thus, the glycosidase regimen can include, in various embodiments, one or more of neuraminidase, galactosidase (e.g., β-Galactosidase), N-acetylgalactosaminidase, fucosidase, glucosidase, N-acetylglucosaminidase, and mannosidase.

In some embodiments, the glycosidase regimen comprises neuraminidase treatment. The neuraminidase therapy may employ a neuraminidase or purified fraction having neuraminidase (sialidase) activity, or an active portion or active derivative thereof. In some embodiments, the neuraminidase is microbial (e.g., bacterial, viral, parasitic, or fungal origin). In still other embodiments, the neuraminidase is mammalian or plant. The neuraminidase may be purified from food materials, including microbes that find use in foods, including baker's yeast and *Lactococcus sp.* and *Lactobacillus sp.* In certain embodiments, the neuraminidase is bacterial. The neuraminidase may be purified or isolated from its natural source, or may be recombinant or synthetic (e.g., chemically synthesized). In some embodiments, the neuraminidase is a γ-Group B neuraminidase, also known as exo-α-sialidase, α-Group B, or acetyl Group B, which cleaves terminal sialic acid residues from carbohydrate moieties on the surfaces of host cells and virus. In some embodiments, the neuraminidase catalyzes the hydrolysis of α-2,3, α-2,6 and/or α-2,8 glycosidic linkages of terminal sialic acid residues in oligosaccharides, glycolipids and colominic acid.

For example, in various embodiments, the neuraminidase is an endo or exo sialidase, for example, catalyzing exo hydrolysis of α-(2→3), α-(2→6), and/or α-(2→8) glycosidic linkages of terminal sialic acid residues, or catalyzing endo hydrolysis of (2→)8)-α-sialosyl linkages in oligo- or poly(sialic) acid. Exemplary neuraminidase agents include any of the well over 100 known neuraminidase enzymes, or active portion or derivative thereof. In some embodiments, the neuraminidase is an enzyme from one or more of *Clostridium perfringes, Arthrobacter ureafaciens, Vibrio cholerae, Salmonella typhimurium,* or *Streptococcus pneumoniae,* or other whose activities are well characterized. Such neuraminidase enzymes may be purified or isolated from its microbial source, or produced recombinantly or synthetically. See Cassidy JT, The Sialic Acids - VI. Purification and properties of sialidase from Clostridium per fringes. J Biol. Chem. 240:9:3501-3506 (1965); Crennell S., et al., Crystal structure of Vibrio cholerae neuraminidase reveals dual lectin-like domains in addition to the catalytic domain. Structure 2:535-544 (1994); Uchida et al., Enzymatic properties of neuraminidases from Arthrobacter ureafaciens. J Biochem. 106:1086-1089 (1979), and these references are hereby incorporated by reference. When in purified form, the neuraminidase is at least 10% of the protein component of the composition, at least 25% of the protein component of the composition, 50% of the protein component of the composition, or at least 75% of the total protein component, or at least 90% of the total protein component, or at least 95% of the total protein component, or at least 99% of the total protein component.

Exemplary amino acid sequences for neuraminidase proteins include those defined by GenBank accession numbers: EIA17609.1, EIA17977.1, NP_561469.1, CAA50436.1, (*Clostridium perfringes*), AAX22758.1, BAD66680.2 (*Arthrobacter ureafaciens*), AAW31751.2, AAA27546.1, AEA78761.1 (*Vibrio cholerae*), 2VWO_A (*Streptococcus pneumoniae*), and AAL19864 (*Salmonella typhimurium*), which are each hereby incorporated by reference. The neuraminidase may comprise an amino acid sequence having at least 70%, 80%, 90%, 95%, or more amino acid sequence identity to one or more of the amino acid sequences defined by EIA17609.1, EIA17977.1, NP_561469.1, CAA50436.1, AAX22758.1, BAD66680.2, AAW31751.2, AAA27546.1, AEA78761.1, 2VWO_A, and AAL19864. Additional neuraminidase enzymes are described in U.S. Patent 8,012,733, U.S. Patent 6,916,916, U.S. Patent. 5,985,859, U.S. Patent 5,830,748, and U.S. Patent 4,071,408, which descriptions are hereby incorporated by reference in their entireties.

Suitable neuraminidases can be obtained from commercial sources. Exemplary neuraminidase enzymes include Sigma Aldrich product numbers N2876, N3001, N5631, N2133 (*Clostridium perfringes),* N7885, N6514 (*Vibrio cholerae*), N3786, and N8271 (*Arthrobacter ureafaciens*).

Preparation of derivatives or mutants of these or other neuraminidase enzymes may be guided by any of the known structures or studies, including those described by: Kirn S et al., Features and applications of bacterial sialidases, Appl Microbiol Biotechnol. 2011, 91(1):1-15; Crennell SJ, et al., Crystal structure of a bacterial sialidase (from Salmonella typhimurium LT2) shows the same fold as an influenza virus neuraminidase. Proc Natl Acad Sci USA 1993 90(21):9852-6; Chavas LM, Crystal structure of the human cytosolic sialidase Neu2: Evidence for the dynamic nature of substrate recognition, J Biol Chem. 2005 280(1):469-75; Xu G et al, Crystal structure of the NanB sialidase from Streptococcus pneumoniae, J Mol Biol. 2008 384(2):436-49; Newstead SL, et al., The structure of Clostridium perfringens Nanl sialidase and its catalytic intermediates, J Biol Chem. 2008 283(14):9080-8; Chan J, et al., Bacterial and viral sialidases: contribution of the conserved active site glutamate to catalysis, Biochemistry 2012 51(1):433-41; Chien CH, et al., Site-directed mutations of the catalytic and conserved amino acids of the neuraminidase gene, napH. of Clostridium perfringes ATCC 10543, Enzyme Microb. Technol. 19(4):267-276 (1996); Christensen and Egebjerg, Cloning, expression and characterization of a sialidase gene from Arthrobacter ureafaciens. Biotechnol. Appl. Biochem. 41:225-231 (2005). These references are each hereby incorporated by reference in their entireties.

Neuraminidase activity can generally be described in terms of units (U), by determining the amount, of sialic acid released from a suitable substrate, under defined conditions (e.g., pH 5.0 and 37°C). An exemplary substrate is NAN-lactose or bovine submaxillary mucin. See Warren L, J Biol. Chem. 234 1971 (1959).

In these and other independent embodiments, the glycosidase regimen comprises galactosidase administration, which is some embodiments is β-galactosidase. Galactosidase may be co-formulated with neuramindase or other enzyme in embodiments involving two or more glycosidase enzymes. Alpha- or Beta-galactosyl residues (including 1→3 linked and 1→4 linked) act as terminal glycosides on mammalian cells, including cancer cells and immune cells, and/or may be penultimate glycosyl residues, and may be linked to terminal sialic acids in some instances. Hakomori, Aberant Glycosylation in Cancer Cell Membranes as Focused on Glycolipids: Overview and Perspectives, Cancer Research 45, 2405-2414 (1985); Dwek and Brooks, Harnesing Changes in Cellular Glycosylation in New Cancer Treatment. Strategies. Current Cancer Drug Targets 4:425-442 (2004). Thus, galactosidase (e.g., β-galactosidase) may be used independently according to the methods described herein, or may be used in conjunction with neuramindase or other glycosidase, which in some embodiments, prevents or slows resialylation or re-capping of glycosyl structures, thus rendering the regimen more effective, and supporting less frequent administrations and/or lower dosing.

Exemplary galactosidases are well known and commercially available. For example, β-Galactosidase may be obtained from *Escherichia coli, Aspergillus oryzae, Kluyveromyces lactis,* and *Streptococcus pneumoniae,* as well as other microbial (e.g., bacterial or fungal) and biological sources, including mammalian sources. For example, a suitable β-galactosidase may be obtained from Sigma-Aldrich catalogue number G5635.

In these and other independent embodiments, the glycosidase regimen comprises N-acetylgalactosaminidase administration. N-acetylgalactosaminidase may be co-formulated with neuramindase or other enzyme in embodiments involving two or more glycosidase enzymes. Hakomori, Aberant Glycosylation in Cancer Cell Membranes as Focused on Glycolipids: Overview and Perspectives, Cancer Research 45, 2405-2414 (1985); Dwek and Brooks, Harnesing Changes in Cellular Glycosylation in New Cancer Treatment Strategies, Current Cancer Drug Targets 4:425-442 (2004). N-acetylgalactosaminidase may be used independently according to the methods described herein, or may be used in conjunction with neuramindase or other glycosidase, which in some embodiments, prevents or slows resialylation or recapping of glycosyl chains.

Exemplary N-acetylgalactosaminidases are well known and commercially available. For example, β-N-acetylgalactosaminidase may be obtained from *Bacillus sp.,* as well as other microbial (e.g., bacterial or fungal) and biological sources, including mammalian sources. For example, a suitable β-N-acetylgalactosaminidase may be obtained from Sigma-Aldrich catalogue number A2464.

In these and other independent embodiments, the glycosidase regimen comprises fucosidase administration, which is some embodiments is α-fucosidase. Fucosidase may be co-formulated with neuramindase or other enzyme in embodiments involving two or more glycosidase enzymes (e.g., co-formulated with galactosidase or N-acetylgalactosaminidase). Fucosyl residues (including α1→2 linked, α1→3 linked, and α1→4 linked) act as terminal glycosides on mammalian cells, including cancer cells and immune cells, and/or may be penultimate glycosyl residues, and may be linked to terminal sialic acids in some instances. Hakomori, Aberant Glycosylation in Cancer Cell Membranes as Focused on Glycolipids: Overview and Perspectives, Cancer Research 45, 2405-2414 (1985); Dwek and Brooks, Harnesing Changes in Cellular Glycosylation in New Cancer Treatment Strategies, Current Cancer Drug Targets 4:425-442 (2004). Thus, fucosidase (e.g., α-fucosidase) may be used independently according to the methods described herein, or may be used in conjunction with neuramindase, which in some embodiments, prevents or slows resialylation re-capping of glycosyl chains, thus rendering the regimen more effective, and supporting less frequent administrations and/or lower dosing.

Exemplary fucosidase enzymes are well known and commercially available. For example, α-fucosidase may be obtained *from Xanthomonas sp.* (e.g., *manihotis*), as well as other microbial (e.g., bacterial or fungal) and biological sources, including mammalian sources. For example, a suitable α-fucosidase may be obtained from Sigma-Aldrich catalogue numbers F3023 and F1924.

In these and other independent embodiments, the glycosidase regimen comprises glucosidase administration, which is some embodiments is α-glucosidase. Glucosidase may be co-formulated with neuramindase or other enzyme in embodiments involving two or more glycosidase enzymes (e.g., co-formulated with neuraminidase, mannosidase, or N-acetylglucosaminidase), Glycosyl residues (including α1→2 linked, α1→3 linked, and α1→4 linked) act as terminal glycosides on mammalian cells, including cancer cells and immune cells, and/or in some instances may be internal glycosyl residues. Hakomori, Aberant Glycosylation in Cancer Cell Membranes as Focused on Glycolipids: Overview and Perspectives, Cancer Research 45, 2405-2414 (1985); Dwek and Brooks, Harnesing Changes in Cellular Glycosylation in New Cancer Treatment Strategies, Current Cancer Drug Targets 4:425-442 (2004). Thus, glucosidase (e.g., α-glucosidase) may be used independently according to the methods described herein, or may be used in conjunction with neuramindase, mannosidase, or other glycosidase, which in some embodiments, prevents or slows resialylation re-capping of glycosyl chains, thus rendering the regimen more effective, and supporting less frequent administrations and/or lower dosing.

Exemplary glucosidase enzymes are well known and commercially available. For example, α-glucosidase may be obtained from *Sacchromyces cerevisiae, Aspergillus niger,* or *Bacillus stearothermophilus,* as well as other microbial (e.g., bacterial or fungal) and biological sources (including food sources such as rice), and including mammalian sources. For example, a suitable α-glucosidase may be obtained from Sigma-Aldrich catalogue numbers G5003, G0660, 70797, 49291, G9259, and G3651.

In these and other independent embodiments, the glycosidase regimen comprises N-acetylglucosaminidase administration, which is some embodiments is β-N-acetylglucosaminidase. N-acetylglucosaminidase may be co-formulated with neuramindase or other enzyme in embodiments involving two or more glycosidase enzymes (e.g., co-formulated with neuraminidase, mannosidase, and/or glucosidase). N-acetylglucosamine residues (including β1→4 linked, β1→6 linked, and others) act as terminal glycosides on mammalian cells, including cancer cells and immune cells, and/or in some instances may be penultimate or internal glycosyl residues. Hakomori, Aberant Glycosylation in Cancer Cell Membranes as Focused on Glycolipids: Overview and Perspectives, Cancer Research 45, 2405-2414 (1985); Dwek and Brooks, Harnesing Changes in Cellular Glycosylation in New Cancer Treatment Strategies, Current Cancer Drug Targets 4:425-442 (2004). Thus, N-acetylglucosaminidase (e.g., β-N-acetylglucosaminidase) may be used independently according to the methods described herein, or may be used in conjunction with neuramindase, mannosidase, or glucosidase, which in some embodiments, prevents or slows resialylation re-capping of glycosyl chains, thus rendering the regimen more effective, and supporting less frequent administrations and/or lower dosing.

Exemplary N-acetylglucosaminidase enzymes are well known and commercially available. For example, β-N-acetylglucosamindase may be obtained from *Streptococcus pneumoniae* and *Canavalia ensiformis,* as well as other microbial (e.g., bacterial or fungal) and biological sources (including food sources), and including mammalian sources. For example, a suitable β-N-acetylglucosaminidase may be obtained from Sigma-Aldrich catalogue numbers A2264 and A6803.

In these and other independent embodiments, the glycosidase regimen comprises mannosidase administration, which is some embodiments is α-mannosidase. Mannosidase may be co-formulated with neuramindase or other enzyme in embodiments involving two or more glycosidase enzymes (e.g., co-formulated with neuraminidase, glucosidase, and/or N-acetylglucosaminidase). Mannosyl residues (including α1→2 linked, α1→3 linked, α1→6 linked, β1→4 linked, and others) act as terminal glycosides on mammalian cells, including cancer cells and immune cells, and/or in some instances may be penultimate or internal glycosyl residues. Hakomori, Aberant Glycosylation in Cancer Cell Membranes as Focused on Glycolipids: Overview and Perspectives, Cancer Research 45, 2405-2414 (1985); Dwek and Brooks, Harnesing Changes in Cellular Glycosylation in New Cancer Treatment Strategies, Current Cancer Drug Targets 4:425-442 (2004). Thus, mannosidase (e.g., α-mannosidase) may be used independently according to the methods described herein, or may be used in conjunction with neuramindase, glucosidase, and/or N-acetylglucosaminidase, which in some embodiments, prevents or slows resialylation re-capping of glycosyl chains, thus rendering the regimen more effective, and supporting less frequent administrations and/or lower dosing.

Exemplary mannosidase enzymes are well known and commercially available. For example, mannosidase may be obtained from *Canavalia enformis* (α) or *Helix pomatia* (β), as well as other microbial (e.g., bacterial or fungal) and biological sources (including food sources), and including mammalian sources. For example, a suitable mannosidase may be obtained from Sigma-Aldrich catalogue numbers M7257 or M9400.

In various embodiments, without wishing to be bound by theory, the glycosidase regimen, which may comprise one or more of neuraminidase, β-galactosidase, α-mannidase, fucosidase (as well as other glycosidase enzymes, including those described herein) converts vitamin D binding protein (also known as group specific component, or Gc), to an effective macrophage activating factor *in vivo,* leading to activation of macrophages. See, for example, U.S. Patent 5,326,749, which is hereby incorporated by reference; Yamamoto et al., J Immunology Vol. 151:2794-2802 (1993). Vitamin D-binding protein, also known as DBP, is an evolutionarily conserved glycoprotein, and is genetically polymorphic. DBP has a relative molecular weight of about 52,000, and normally constitutes about 0.5% of the plasma protein. The proper dose and regimen of glycosidase as described herein deglycosylates DBP *in vivo,* leading to effective, consistent, and chronic *in vivo* macrophage activation and anti-tumor activity.

Administration of effective amounts of formulated glycosidase to a human or animal aids in the prevention or elimination of cancer symptoms through modulation of the immune function and/or direct action on the cancer cells, or other tissues or cells involved in the pathology, as well as deglycosylation of serum proteins. The glycosidase enzymes are administered in various embodiments at a dose and frequency so as to exhibit a reduction in cancer symptoms or pathology, without impacting normal cellular functions. The dose and/or frequency of administration in some embodiments is a dose and/or frequency that does not cause prolonged joint discomfort or malaise (e.g., a general feeling of discomfort, or arthritic sensation in one or more joints). Where joint or general discomfort is experienced by the patient, the patient may adjust the dose or frequency of administration until the discomfort subsides or normalizes. For example, where the patient experiences discomfort, the patient may skip one, two, or three days of dosing, and/or subtract one or two daily doses from the regimen, and/or increase the timing between doses, until the discomfort subsides or normalizes. Thus, the patient finds the highest dose and/or frequency of administration that induces no prolonged joint discomfort, or minimal discomfort. In some embodiments where the amount of dose is controllable, for example using a metered dose applicator, the dose may be reduced but the schedule maintained. Thus, each patient can tailor the dose as needed given the state of the patient's unique biology, disease or immune system condition, by finding the highest dose/frequency that does not induce prolonged joint discomfort or malaise. In practice, the glycosidase formulation may be administered at less than approximately 10⁻² or less than about 10⁻³ mg per dosage unit to a human or animal. In certain embodiments, the glycosidase(s) are administered at between approximately 10⁻³ mg to 10⁻⁸ mg. In still other embodiments, the dose of glycosidase is between approximately 10⁻³ mg and 10⁻⁷ mg, 10⁻³ mg and 10⁻⁶ mg, 10⁻³ mg and 10⁻⁵ mg, or is approximately 10⁻⁴ mg. In some embodiments, the total daily dose does not exceed about 10⁻³ mg per subject, or in some embodiments, does not exceed from about 5 x 10⁻³ to 10⁻⁴ mg.

While certain glycosidases, including neuraminidases, can have a tendency to form homodimers (e.g., trimers, tetramers), in various embodiments the glycosidase(s) are formulated (e.g., diluted) to be present as a monomer and/or dimer, with substantially no higher aggregates as determinable by size exclusion chromatography (SEC).

The glycosidase(s) may be formulated as an aqueous formulation, including for sublingual, nasal, or buccal delivery. In some embodiments, the aqueous formulation comprises saline. In some embodiments, the formulation has the ionic strength of from about 0.5 to about 2% saline, such as the ionic strength of about 0.9% saline. In some embodiments, the glycosidase(s) are formulated in normal saline (e.g., about 0.9% saline). Other conventional carriers for sublingual, nasal, or buccal delivery may also be employed. The glycosidase(s) may be further formulated with a preservative, which may be an aromatic or phenolic preservative. For example, the preservative in some embodiments is phenol. For example, in some embodiments neuraminidase, optionally with other glycosidases, is formulated in 0.05 to 0.5% phenol, or comparable amounts of similar acting preservative, for example. In some embodiments, the activity of the neuraminidase and potentially other glycosidases is increased by the presence of phenol, such as at least 0.2%, 0.3%, or 0.4% phenol. In some embodiments the neuraminidase (e.g., Sigma Aldrich catalogue numbers N2876, N3001, N5631, N2133, N7885, N6514, N3786, N8271) is incubated in a solution containing from about 0.2% to about 1% phenol (e.g., from 0.2 to 0.6% phenol, or about 0.4% phenol), and then diluted to or brought to the final formulation, which may contain from 0.05% to about 0.2% phenol. In some embodiments, such "activation" of the neuraminidase allows the active agent to be administered in lower doses to avoid immune targeting, while maintaining the proper level of activity.

For illustration, neuraminidase and optionally with other glycosidase enzyme(s), can be mixed with 0.9% saline, and filter sterilized, and allowed to stand at room temperature for from 10 minutes to five hours (e.g., about 30 minutes to about three hours). After the incubation at room temperature, phenol saline is added to give a final phenol concentration of about 0.1% in 0.9% saline solution. The solution is stored at 4° C.

Alternatively, neuraminidase and optionally other glycosidase enzyme(s) is mixed with about 0.4% phenol saline. This solution is filter sterilized, and allowed to stand at room temperature for from 5 minutes to about 5 hours (e.g., about 30 minutes, about one hour, or about three hours). After the incubation at room temperature, the final concentration is brought to about 0.1% phenol, 0.9% saline. The solution is stored at 4° C.

The glycosidase formulation may be administered by a variety of routes, including sublingual, nasal, port, subdermal, gavage, intraocular, intravenous, intramuscular, subcutaneous, transdermal, and buccal. In various embodiments, the glycosidases are administered sublingually. In some embodiments in which the glycosidase is administered sublingually, the neuraminidase is held under the tongue for from about one to about five minutes, and preferably for about 3, about 4, or about 5 minutes. The patient should refrain from speaking during this time. The patient should not eat or drink within 15 minutes of administration.

In accordance with aspects of the invention, regimens of glycosidase enzyme(s) are administered on average from 2 to 6 times per day for at least two weeks or at least one month, especially for the immune compromised or advanced cases. The daily administrations should be substantially evenly spaced, but in various embodiments are spaced by about 15 minutes to 5 hours. For example, doses may be spaced by about 15 minutes, about 30 minutes, about 1 hour, about 2 hours, or about 3 hours. For example, the glycosidase formulation may be administered on average from 2 to 8 times per day for at least about two months, at least about four months, or at least about five months, or at least about six months. In some embodiments, the glycosidase formulation is administered about 2, about 3, or about 4 times per day over at least one month, two months, three months, four months, five months, or six months. Generally, the glycosidase formulation is administered at a dose and frequency so as to be effective in reducing the cancer pathology or stimulating the immune system, without exhibiting substantial joint discomfort (e.g., arthritic sensation) or malaise. Where the patient does experience joint or general discomfort, the patient adjusts the dose and/or frequency (e.g., skips one, two or three days of neuraminidase dosing, or reduces the daily dose by one or two administrations), until the discomfort subsides or normalizes. Thus, the administration regimen is suspended during times of joint discomfort in some embodiments.

For example, the first day of treatment may begin with about eight doses, the first three to five taken in the first one or two hours, with the remainder approximately evenly spaced throughout the day. The patient may then be treated with about four doses per day, with periodic monitoring of the malignancy. Even where the malignancy is undetectable, the patient may remain on a regimen of 2 to 7 doses per day, as adjusted from time to time based on the appearance of joint discomfort or malaise.

Therapy may be initiated as described above, in preparation for chemotherapy, radiation therapy, or surgical treatment, or alternatively, during the treatment to increase effectiveness, and/or after the treatment for recuperative purposes. However, in some embodiments, the glycosidase regimen is an alternative to these conventional therapies. In some embodiments, the patient is subsequently treated chronically with about one dose per day, for at least about six months, or at least about one year, or at least about two years, or at least about five years, or more, or is selected or prescribed for such chronic treatment. This subsequent chronic treatment in some embodiments is with the absence of chemotherapeutic or other therapy to reduce the likelihood of recurrence or disease progression. Chronic glycosidase treatment, for example, to prevent cancer or prevent cancer recurrence or progression to locally advanced disease, may be administered 1 or 2 times per day.

In some embodiments, the patient is instructed to monitor joint stiffness or malaise. Such conditions suggest that glycosidase treatment should be adjusted. The adjustment may include skipping one or two days or up to one week of dosing, or alternatively lowering the dose by one or two administrations per day, until the symptoms clear. Other molecular assays could be used to the same effect, although joint stiffness or discomfort provides an ease of patient compliance. Thus, over the course of the regimen, the glycosidase dose can be easily adjusted per patient, and thus maintained chronically for optimal care.

In various embodiments where the glycosidase regimen is provided for treatment of early stage cancer prior to other intervention, or in other instances to increase a more advanced patient's fitness for other therapy, the length of the glycosidase regimen can be determined by improvement or normalization of a biomarker panel or reduction in one or more tumor markers, which may be determined in blood or urine, for example. Such markers may be evaluated about weekly or about biweekly, or about monthly, and the glycosidase regimen continued as long as improvement or normalization of the marker(s) is observed. Surgery, chemotherapy, and/or radiation therapy is postponed, and only initiated to the extent that the glycosidase therapy by itself is not enough to eliminate the cancer.

In some embodiments, the glycosidase therapy is continued for as long as benefits can be observed via one or more tumor markers or immune parameters.

For example, in some embodiments the cancer is liver cancer or a germ cell cancer, and the response to glycosidase therapy is monitored by Alpha-fetoprotein (AFP) levels in blood.

In some embodiments, the cancer is multiple myeloma, chronic lymphocytic leukemia, or lymphoma, and the response to glycosidase therapy is monitored by Beta-2-microglobulin levels (B2M) or immunoglobulin levels in blood or urine, or monitored by BCR-ABL detection.

In some embodiments, the cancer is choriocarcinoma or testicular cancer, and the response to glycosidase therapy is monitored by Beta-human chorionic gonadotropin (Beta-hCG) levels in blood or urine.

In some embodiments, the cancer is breast cancer, and the response to glycosidase therapy is monitored by CA15-3/CA27.29 levels in blood.

In some embodiments, the cancer is pancreatic cancer, gallbladder cancer, bile duct cancer, or gastric cancer, and the response to glycosidase therapy is monitored by CA19-9 levels in blood.

In some embodiments, the cancer is ovarian cancer, and the response to glycosidase therapy is monitored by CA-125 levels in blood.

In some embodiments, the cancer is medullary thyroid cancer, and the response to glycosidase therapy is monitored by calcitonin levels in blood.

In some embodiments, the cancer is breast cancer or colorectal cancer, and the response to glycosidase therapy is monitored by Carcinoembryonic antigen (CEA) levels in blood or presence or level of urokinase plasminogen activator (uPA) and plasminogen activator inhibitor (PAI-1) in the tumor.

In some embodiments, the cancer is a. neuroendocrine tumor, and the response to glycosidase therapy is monitored by Chromogranin A (CgA) levels in blood.

In some embodiments, the cancer is bladder cancer, and the response to glycosidase therapy is monitored by Fibrin/fibrinogen levels or Nuclear matrix protein 22 levels in urine.

In some embodiments, the cancer is ovarian, and the response to glycosidase therapy is monitored by HE4 levels in blood.

In some embodiments, the cancer is a germ cell cancer, and the response to glycosidase therapy is monitored by lactate dehydrogenase levels in blood.

In some embodiments, the cancer is prostate cancer, and the response to glycosidase therapy is monitored by prostate-specific antigen (PSA) levels in blood.

In some embodiments, the cancer is thyroid cancer, and the response to glycosidase therapy is monitored by thyroglobulin levels in the tumor.

Basic immune or blood parameters that can be monitored for improvement or normalization including one or more of: white blood cells, red blood cells or hematocrit or red blood cell indices, hemoglobin or mean corpuscular hemoglobin (MCH or MCHC), neutrophil granulocytes, lymphocytes, monocytes, eosinophil granulocytes, basophil granulocytes, platelets or mean platelet volume (MPV).

In still other aspects of the invention there is provided a pharmaceutical composition comprising a delivery vehicle for administering a single glycosidase dose upon demand, and where the vehicle contains a full glycosidase regimen of at least 50 doses, or at least 100 doses, at least 150 doses, or at least 200 doses. Each dose of glycosidase administered is an amount of up to about 10⁻² mg glycosidase and pharmaceutically inert ingredients as already described. The pharmaceutical composition may comprise at least two of neuraminidase, galactosidase, N-acetylgalactosaminidase, fucosidase, glucosidase, N-acetylglucosaminidase, and mannosidase, and a pharmaceutically-acceptable excipient. For example, the composition may comprise neuraminidase and β-galactosidase. The glycosidases may be present at, collectively, between about 10⁻³ mg to 10⁻⁸ mg, or according to the doses disclosed above. The composition may be formulated for a variety of administration routes as disclosed herein, including sublingual delivery.

In various embodiments, the treatment regimen involves the partitional administration of an amount not to exceed approximately 10⁻³ mg of glycosidase, although, in certain cases, the total amount of glycosidase administered in any one day may exceed this limit.

The glycosidase formulation can be administered in a variety of routes and forms. For example, the glycosidase can be administered as a solid where the enzymes are embedded or admixed in a biodegradable or bioerodable matrix. The matrix can be a time release matrix. These matrices are well known to those of ordinary skill in the art. The glycosidase can be administered by injection or by sublingual route. In one embodiment, the vehicle is an aqueous solution that is contained within an inert, container. In another variation, the composition is in the form of a suppository. The liquid form of the composition can be injected subcutaneously, intramuscularly or intravenously. In addition, the composition can be administered through the mucosal membranes such as nasal membranes.

In certain embodiments, the glycosidase composition is administered via a drug applicator, the applicator comprising at least 100 doses of the composition, or at least 150 doses, or at least 200 doses. In various embodiments, the applicator is for sublingual, nasal, transdermal, time release sub-dermal, intraocular, gavage, port, subcutaneous, oral, or buccal delivery. For example, the applicator is for sublingual delivery.

In some embodiments, the applicator delivers a metered dose, that can be adjusted by the patient as needed.

The applicator preferably dispenses doses in a manner that maintains aseptic conditions of the remaining doses. By way of non-limiting examples, the applicator can be any of those that are described in US Patents 4,830,284; 4,565,302; 5,011,046; 5,147,087; 5,893,484; 6,877,672; 6,886,556, and 7,201,296, which are each hereby incorporated by reference in their entireties. For instance, the applicator can be an atomizing or dosing pump, which can ensure that the medium present in the area between the pump cylinder and the discharge opening does not dry or is not otherwise altered by ambient influences. See US Patent 4,830,284 which is hereby incorporated by reference. In some embodiments, the applicator employs a 0.2µm filter to maintain aseptic contents. Additionally, the applicator can dispense doses in a single-stroke discharge. Such applicators are described in US Patent 5,893,484, which is hereby incorporated by reference. The applicator may be configured for nasal delivery, dermal delivery, throat delivery, or sublingual delivery. In some embodiments, the applicator allows for an actuatable dosing mechanism, which permits monitoring of precise doses and therefore largely eliminates incorrect dosing with respect to the number of doses and/or the duration dosing. See US Patent 4,565,302, which is hereby incorporated by reference. In some embodiments, the applicator delivers a dose in from 50 to 100 µl, such as the applicators described in, for example, US Patent 6,886,556. which is hereby incorporated by reference.

### Disclosed Items

1. A method for improving outcome or rate of response in cancer treatment, comprising administering a regimen of immunotolerant glycosidase to one or more patients undergoing treatment for cancer to remove an effective amount of glycosyl residues from cancer cells and/or immune cells and/or serum proteins.
2. The method of item 1, wherein the glycosidase(s) facilitate revealing of antigen on malignant cells and/or facilitate immune cascades.
3. The method of item 2, wherein the glycosidase(s) comprises one or more of neuraminidase, galactosidase, N-acetylgalactosaminidase, fucosidase, glucosidase, N-acetylglucosaminidase, and mannosidase.
4. The method of item 2 or 3, wherein the glycosidase(s) comprise at least two or three glycosidase enzymes.
5. The method of item 4, wherein the glycosidase enzymes comprise neuraminidase and/or β-galactosidase.
6. The method of any one of items 1 to 5, wherein the one or more patients do not undergo a molecular analysis of the tumor or cancer cells.
7. The method of any one of items 1 to 5, wherein the one or more patients do not undergo chemosensitivity testing of the tumor or cancer cells.
8. The method of any one of items 1 to 5, wherein the patient has a poor prognosis for survival.
9. The method of any one of items 1 to 5, wherein the cancers each comprise a solid tumor.
10. The method of item 8 or 9, wherein the cancer is heavily sialylated.
11. The method of item 10, wherein the cancer is one or more of lung cancer including SCLC and NSCLC, mesothelioma, brain cancer, glioblastoma, head and neck cancer, esophageal cancer, breast cancer, lymphoma, prostate cancer, pancreatic cancer, liver cancer, stomach cancer, colon or colorectal cancer, ovarian cancer, endometrial cancer, cervical cancer, testicular cancer, and melanoma.
12. The method of any one of items 9 to 11, wherein the cancer is stage I or is premetastatic.
13. The method of any one of items 9 to 11, wherein the cancer is stage II or III.
14. The method of any one of items 9 to 11, wherein the cancer is stage IV, or is metastatic.
15. The method of any one of items 9 to 11, wherein the cancer is non-resectable.
16. The method of any one of items 9 to 11, wherein the cancer is multidrug resistant.
17. The method of any one of items 1 to 5, wherein the cancer is a leukemia.
18. The method of item 17, wherein the leukemia is chronic myelogenous leukemia (CML) or acute lymphoblastic leukemia (ALL), and which may be drug resistant.
19. The method of any one of items 1 to 18, wherein the patient is in remission.
20. The method of any one of items 1 to 18, wherein the cancer is a recurrence.
21. The method of any one of items 1 to 20, wherein the glycosidase regimen is administered to prepare the patient for radiation therapy.
22. The method of any one of items 1 to 21, wherein the glycosidase regimen is administered after radiation therapy.
23. The method of any one of items 1 to 22, wherein the glycosidase is administered during the radiation treatment regimen.
24. The method of any one of items 1 to 16 and 19 to 23, wherein the glycosidase regimen is administered prior to tumor resection, optionally with neoadjuvant chemotherapy.
25. The method of any one of items 1 to 16 and 19 to 23, wherein the glycosidase regimen is administered after tumor resection, optionally with adjuvant chemotherapy.
26. The method of any one of items 1 to 25, wherein the patient(s) receive a chemotherapy selected from an anthracyclin, taxol or taxoid, vinca alkaloid, alkylating agent, intercalating agent, immunomodulator or immunoconjugate, kinase inhibitor, and nitrogen mustard.
27. The method of any one of items 1 to 26, wherein the patient(s) receive a topoisomerase inhibitor (I or II), apoptosis inducer, protease inhibitor, microtubule inhibitor, mitotic inhibitor, an antimetabolite, signal transduction inhibitor, estrogen receptor inhibitor, EGFR inhibitor, Her2 inhibitor, or an aromatase inhibitor.
28. The method of any one of items 1 to 27, wherein the patient(s) receive one or more of daunorubicin, doxorubicin, epirubicin, idarubicin, adriamycin, vincristine, carmustine, cisplatin, 5-fluorouracil, tamoxifen, prodasone, sandostatine, mitomycin C, foscarnet, paclitaxel, docetaxel, gemcitabine, fludarabine, carboplatin, leucovorin, tamoxifen, goserelin, ketoconazole, leuprolide flutamide, vinblastine, vindesine, vinorelbine, camptothecin, topotecan, irinotecan hydrochloride, etoposide, mitoxantrone, teniposide, amsacrine, merbarone, piroxantrone hydrochloride, methotrexate, 6-mercaptopurine, 6-thioguanine, cytarabine (Ara-C), trimetrexate, acivicin, alanosine, pyrazofurin, pentostatin, 5-azacitidine, 5-azacitidine, 5-Aza-5-Aza-2'-deoxycytidine, adenosine arabinoside (Ara-A), cladribine, ftorafur, UFT (combination of uracil and ftorafur), 5-fluoro-2'-deoxyuridine, 5-fluorouridine, 5'-deoxy-5-fluorouridine, hydroxyurea, dihydrolenchiorambucil, tiazofurin, oxaliplatin, melphalan, thiotepa, busulfan, chlorambucil, plicamycin, dacarbazine, ifosfamide phosphate, cyclophosphamide, pipobroman, 4-ipomeanol, dihydrolenperone, spiromustine, geldenamycin, cytochalasins, depsipeptide, 4'-cyano-3-(4-(e.g., Zoladex) and 4'-cyano-3-(4-fluorophenylsulphonyl)-2-hydroxy-3-methyl-3'-(trifluorometh-yl)propionanilide.
29. The method of any one of items 1 to 28, wherein the patient(s) receive one or more of anti-Her2/neu antibodies such as Herceptin, an anti-EGFR antibody such as Erbitux, a growth factor receptor antibody such as Avastin, a small molecule inhibitor such as Tarceva, Iressa, or sunitinib), or anti-CD20 such as Rituxan.
30. The method of item 29, wherein the patient(s) receive erlotinib, gefitinib, lapatinib, cetuximab, panitumumab, or imatinib.
31. The method of item 26, wherein the patient receives a stomatotoxic chemotherapy.
32. The method of item 31, wherein the stomatotoxic chemotherapy is one or a combination of 5-fluorouracil, methotrexate, cytarabine, cisplatin, carboplatin, paclitaxel, docetaxel, doxorubicin, and etoposide.
33. The method of any one of items 1 to 23, wherein the glycosidase regimen is administered as an alternative to chemotherapy.
34. The method of item 33, wherein the patient is a poor candidate for chemotherapeutic treatment.
35. The method of any one of items 24 to 32, wherein the glycosidase regimen is administered prior to a chemotherapy cycle.
36. The method of any one of items 21 to 29, wherein the glycosidase regimen is administered between chemotherapy cycles.
37. The method of any one of items 24 to 32, wherein the glycosidase regimen is administered after the completion of chemotherapy.
38. The method of item 1, wherein the patient receives a bone marrow transplant, and the glycosidase regimen is provided prior to, and/or after transplant.
39. The method of any one of items 1 to 38, wherein the glycosidase dose and/or frequency of administration does not cause prolonged joint discomfort or malaise.
40. The method of item 39, wherein the glycosidase(s) is not administered at times of joint discomfort or malaise.
41. A method for treating a tumor that is resistant to chemotherapy treatment, comprising, administering an immune tolerant glycosidase regimen to the patient.
42. The method of item 41, wherein the glycosidase regimen is administered after unsuccessful chemotherapy treatment.
43. The method of item 41, wherein the cancer is a recurrence after chemotherapeutic treatment.
44. The method of item 41, wherein the cancer has one or more biomarkers of resistant cancer.
45. The method of any one of items 41 to 44, wherein the glycosidase dose and/or frequency of administration does not cause prolonged discomfort.
46. The method of item 45, wherein the glycosidase(s) is not administered at times of joint discomfort or malaise.
47. A method of preventing cancer in a patient predisposed to develop cancer, comprising, administering an immunotolerant glycosidase signaling regimen to the patient.
48. The method of item 47, wherein the patient is characterized by genetic predisposition, which is optionally breast cancer, colon cancer, or skin cancer.
49. The method of item 48, wherein the patient has had at least two prior incidences of a cancer.
50. The method of any one of items 1 to 49, wherein the glycosidase is administered by a route selected from sublingual, nasal, port, subdermal, gavage, intraocular, intravenous, intramuscular, subcutaneous, transdermal, and buccal.
51. The method of item 50, wherein the glycosidase is administered sublingually.
52. The method of item 50, wherein the glycosidase(s) is/are of biological origin, or a derivative or active moiety thereof, which maintains immune cascading activity.
53. The method of item 50, wherein the glycosidase is from a biological source or is recombinant or synthetic.
54. The method of any one of items 1 to 53, wherein the glycosidase(s) is/are administered on average from 2 to 6 times per day for at least one week, at least one month.
55. The method of item 54, wherein the glycosidase(s) is/are administered on average from 2 to 6 times per day for at least two months, at least four months, or at least six months.
56. The method of item 54, wherein the patient is subsequently treated chronically with about one dose per day, for at least six months, or at least one year, or at least two years, or at least five years, or more.
57. The method of any one of items 50 to 56, wherein the glycosidase dose or frequency of dosing is adjusted based on discomfort, such as joint discomfort.
58. A pharmaceutical composition comprising at least two of neuraminidase, galactosidase, N-acetylgalactosaminidase, fucosidase, glucosidase, N-acetylglucosaminidase, and mannosidase, and a pharmaceutically-acceptable excipient.
59. The composition of item 58, wherein the composition comprises neuraminidase and β-galactosidase.
60. The composition of item 58 or 59, wherein the glycosidases are present at, collectively, between approximately 10⁻³ mg to 10⁻⁸ mg.
61. The composition of item 60, wherein the composition is formulated for sublingual delivery.
62. A drug applicator for administration of a glycosidase composition of any one of items 58 to 61, the applicator comprising at least 100 doses of the glycosidase composition.
63. The drug applicator of item 62, wherein the applicator is for sublingual, nasal, transdermal, time release sub-dermal, intraocular, gavage, port, subcutaneous, oral, or buccal delivery.
64. The drug applicator of item 62, wherein the applicator is for sublingual delivery.
65. The drug applicator of any one of items 62 to 65, wherein the applicator dispenses doses in a manner that maintains aseptic conditions of the remaining doses.
66. The drug applicator of item 65, wherein the applicator delivers a dose in from 50 to 100 µl.

## Claims

1. A glycosidase composition for use in improving outcome or rate of response in patients undergoing treatment for cancer, wherein the composition comprises *Clostridium perfringens* neuraminidase, and optionally one or more of β-galactosidase, N-acetylgalactosaminidase, fucosidase, glucosidase, N-acetylglucosaminidase, and mannosidase.

2. The glycosidase composition for the use of claim 1, wherein the glycosidase(s) comprise at least three glycosidase enzymes.

3. The glycosidase composition for the use of claim 1, wherein the glycosidase enzymes comprise β-galactosidase.

4. The glycosidase composition for the use of claim 1, wherein the glycosidase composition comprises NanI.

5. The glycosidase composition for the use of any one of claims 1 to 4, wherein the composition is to be administered after radiation therapy.

6. The glycosidase composition for the use of any one of claims 1 to 5, wherein the composition is to be administered prior to tumor resection, with neoadjuvant chemotherapy.

7. The glycosidase composition for the use of any one of claims 1 to 5, wherein the composition is to be administered after tumor resection, with adjuvant chemotherapy.

8. The glycosidase composition for the use of any one of claims 1 to 4, wherein the composition is to be administered between chemotherapy cycles, or after the completion of chemotherapy.

9. The glycosidase composition for the use of claim 1, wherein the patient receives a bone marrow transplant, and the glycosidase regimen is to be provided prior to, and/or after transplant.

10. The glycosidase composition for the use of any one of claims 1 to 9, wherein the glycosidase composition is not to be administered at times of joint discomfort.

11. The glycosidase composition for the use of claim 1, wherein the cancer is breast cancer, colon cancer, or skin cancer.

12. The glycosidase composition for the use of claim 11, wherein the patient has had at least two prior incidences of a cancer.

13. The glycosidase composition for the use of any one of claims 1 to 12, wherein the glycosidase is to be administered by a route selected from sublingual, nasal, port, subdermal, gavage, intraocular, intravenous, intramuscular, subcutaneous, transdermal, and buccal.

14. The glycosidase composition for the use of any one of claims 1 to 13, wherein the composition is to be administered on average from 2 to 6 times per day for at least one month.

15. The glycosidase composition for the use of claim 14, wherein the patient is subsequently to be treated chronically with about one dose per day for at least six months.

16. The glycosidase composition for the use of any one of claims 1 to 15, wherein the composition contains from 10⁻³ to 10⁻⁸ mg of glycosidase.
